(19)

Europäisches
Patentamt
European
Patent Office
Office européen
des brevets

(11) **EP 2 216 048 A1**

(12) **EUROPEAN PATENT APPLICATION**
published in accordance with Art. 153(4) EPC

(43) Date of publication:
**11.08.2010 Bulletin 2010/32**

(21) Application number: **08851103.5**

(22) Date of filing: **18.11.2008**

(51) Int Cl.:
*A61K 47/10* (2006.01)          *A61K 9/16* (2006.01)
*A61K 47/38* (2006.01)

(86) International application number:
**PCT/JP2008/070888**

(87) International publication number:
**WO 2009/066644 (28.05.2009 Gazette 2009/22)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **19.11.2007 JP 2007299546**

(71) Applicant: **Freund Corporation
Tokyo 163-6034 (JP)**

(72) Inventor: **TAKAHASHI, Terumi
Hamamatsu-shi
Shizuoka 431-2103 (JP)**

(74) Representative: **Raggers, René John
Exter Polak & Charlouis B.V.
P.O. Box 3241
NL-2280 GE Rijswijk (NL)**

(54) **SPHERICAL PARTICLE AND METHOD FOR PRODUCING THE SAME**

(57)     A spherical particle of the present invention contains a sugar alcohol and a crystalline cellulose and/or powdered cellulose, wherein the mass ratio between the sugar alcohol and the crystalline cellulose and/or powdered cellulose is within a range from 50:50 to 90:10, the particle size is within a range from 75 to 250 $\mu$m, the sphericity is not less than 0.8, and the bulk density is not less than 0.6 g/ml. Further, a method for producing the spherical particle of the present invention includes a granulation step of rolling a sugar alcohol having an average particle size of not more than 40 $\mu$m and a crystalline cellulose and/or powdered cellulose having an average particle size of not more than 50 $\mu$m while spraying a liquid thereon.

EP 2 216 048 A1

**Description**

TECHNICAL FIELD

The present invention relates to a spherical particle, the surface of which is used for layering with an orally ingestible active substance, and also relates to a method for producing such a spherical particle.
Priority is claimed on Japanese Patent Application No. 2007-299546, filed November 19, 2007, the content of which is incorporated herein by reference.

BACKGROUND ART

As humans age, the strength of the swallowing action tends to deteriorate, and the ingestion of typical tablets and capsules can become difficult. Accordingly, these typical tablets and capsules may result in reduced patient compliance. In order to improve patient compliance, in recent years, capsules have been reduced in size, and research relating to orally rapid disintegrating tablets has been actively pursued, with large numbers of actual products now available commercially. An orally rapid disintegrating tablet refers to a tablet that disintegrates rapidly in the mouth, thereby facilitating ingestion.
The term "capsules" includes capsules in which particles prepared by coating an active substance or a film-like base material onto inactive spherical particles are packed inside a hard capsule. When a capsule is produced using this type of method, in order to enable the size of the capsule to be reduced without reducing the amount of the active substance, it is desirable that the spherical particles are produced with a small particle size in order to reduce their packing volume.
Furthermore, a variety of orally rapid disintegrating tablets prepared using all manner of production methods are currently available commercially. One example of an orally rapid disintegrating tablet formulation is a tablet in which an active substance is coated onto a spherical particle, and the resulting medicated spherical particle with a film-like base material coated thereon is incorporated within the tablet formulation. In this case, if the particle size of the medicated spherical particle is too large, then an unpleasant gritty sensation may occur upon disintegration of the formulation within the mouth, and therefore a spherical particle of smaller size is preferred.
For example, Patent Document 1 discloses a spherical particle formed solely from a powdered cellulose that is inert relative to the active substance (medication), and a spherical particle formed by incorporating a water-soluble material such as lactose within the powdered cellulose.
Further, Patent Document 2 discloses a granular agent having a high degree of hardness and superior disintegration properties, which is produced in an extruder using a crystalline cellulose having a 50% cumulative volume average particle size of not more than 10 $\mu$m.
Moreover, Patent Document 3 discloses a spherical particle having a tapping apparent density of not less than 0.65 g/ml and a sphericity of not less than 0.7, which is prepared by controlling the average polymerization degree of a crystalline cellulose.

[Patent Document 1]
Japanese Unexamined Patent Application, First Publication No. Hei 9-295947
[Patent Document 2]
Japanese Unexamined Patent Application, First Publication No. Hei 9-20689
[Patent Document 3]
Japanese Unexamined Patent Application, First Publication No. Hei 7-173050

DISCLOSURE OF INVENTION

PROBLEMS TO BE SOLVED BY THE INVENTION

The spherical particle formed solely from a powdered cellulose disclosed in Patent Document 1 has a high degree of hardness and is resistant to abrasion. However, a problem arises in that the adsorption capacity of the cellulose tends to cause a reduction in the bioavailability (the proportion of the administered medication that can be utilized within the body). As a result, the Patent Document 1 also discloses a method of incorporating cellulose and a water-soluble material within the spherical particle. However, the lactose that is used as the water-soluble material in Patent Document 1 may undergo an interaction such as a Maillard reaction with the active substance (medication) that is incorporated within, or coated onto, the particle during drug preparation. In other words, this spherical particle has the problem of not offering general versatility for use with all manner of active substances.
Furthermore, because the granular agent disclosed in Patent Document 2 is granulated using an extruder, the particle size tends to be large, and there tends to be considerable variation in the particle shape. Accordingly, incorporating this granular agent in a large amount during drug preparation tends to be difficult.

Moreover, the average particle size of the spherical particles reported in the examples disclosed in Patent Document 3 is within a range from 200 to 500 $\mu$m, meaning these spherical particles tend to have too large a particle size for use within orally rapid disintegrating tablets.

The present invention has been developed in light of the above circumstances, and has an object of providing a very fine spherical particle that offers the advantages of a high degree of versatility relative to active substances, meaning there are no restrictions on the active substances that can be used during drug preparation, favorable resistance to abrasion, and minimal reduction in bioavailability, as well as a method for producing such a spherical particle.

MEANS TO SOLVE THE PROBLEMS

A spherical particle according to the present invention contains a sugar alcohol and a crystalline cellulose and/or powdered cellulose, wherein the mass ratio between the sugar alcohol and the crystalline cellulose and/or powdered cellulose is within a range from 50:50 to 90:10, the particle size is within a range from 75 to 250 $\mu$m, the sphericity (minor axis / major axis) is not less than 0.8, and the bulk density is not less than 0.6 g/ml.

Furthermore, the sugar alcohol is preferably D-mannitol, xylitol, D-sorbitol or erythritol, and of these, D-mannitol is the most desirable.

A method for producing a spherical particle according to the present invention includes a granulation step of rolling a sugar alcohol having an average particle size of not more than 40 $\mu$m and a crystalline cellulose and/or powdered cellulose having an average particle size of not more than 50 $\mu$m while spraying a liquid thereon. The sugar alcohol is preferably in a ground form.

Moreover, in the above granulation step, an intermediate treatment is preferably conducted in which the rolling is performed while the spraying of the liquid is halted.

EFFECT OF THE INVENTION

The present invention yields a very fine spherical particle that offers the advantages of a high degree of versatility, meaning there are no restrictions on the active substances that can be used during drug preparation, favorable resistance to abrasion, and minimal reduction in bioavailability. Furthermore, the production method of the present invention enables a spherical particle having these properties to be produced with good yield without performing complex steps.

BEST MODE FOR CARRYING OUT THE INVENTION

A detailed description of preferred embodiments of the present invention is presented below.

Spherical particle

The spherical particle of the present invention contains a sugar alcohol and a crystalline cellulose and/or powdered cellulose, wherein the mass ratio between the sugar alcohol and the crystalline cellulose and/or powdered cellulose is within a range from 50:50 to 90:10, the particle size is within a range from 75 to 250 $\mu$m, the sphericity is not less than 0.8, and the bulk density is not less than 0.6 g/ml.

(Sugar alcohol)

Examples of the sugar alcohol include D-mannitol, xylitol, D-sorbitol, erythritol, lactitol and maltitol. Of these, the use of D-mannitol, xylitol, D-sorbitol or erythritol is preferred. Moreover, the use of D-mannitol is the most desirable as it is chemically stable and contains almost no free water, meaning interactions with the active substance are minimal.

(Crystalline cellulose, powdered cellulose)

The crystalline cellulose and/or powdered cellulose (hereafter referred to jointly as "cellulose") is chemically inert, and because it has a high level of water absorption, also exhibits excellent plastic deformation properties. Accordingly, by using the cellulose during within the spherical particle formulation, a spherical particle with a high degree of versatility relative to active substances can be obtained, meaning no restrictions need be placed on the active substances that can be used during drug preparation. Furthermore, using the cellulose within the spherical particle formulation tends to facilitate formation of a spherical shape during the granulation step.

(Spherical particle)

The mass ratio between the sugar alcohol and the cellulose within the spherical particle is within a range from sugar alcohol : cellulose = 50:50 to 90:10, and is preferably from 50:50 to 80:20 (wherein the combination of the sugar alcohol and the cellulose totals 100).
Provided the proportion of the sugar alcohol is at least 50% by mass (and the proportion of the cellulose is not more than 50% by mass), the amount of medication adsorbed by the cellulose during preparation of the spherical particle can be reduced. Accordingly, any reduction in the bioavailability caused by the spherical particle tends to be suppressed. On the other hand, if the proportion of the sugar alcohol exceeds 90% by mass (and the proportion of the cellulose is less than 10% by mass), then the resulting granulated particle tends to suffer from both low sphericity and a low degree of hardness, and the yield of the desired spherical particle tends to decrease.

A spherical particle of the present invention containing the components described above has a particle size within a range from 75 to 250 $\mu$m, and preferably from 75 to 150 $\mu$m.
Provided the particle size is not less than 75 $\mu$m, aggregation can be reduced during drug preparation, resulting in more favorable handling. On the other hand, if the particle size exceeds 250 $\mu$m, then, for example, in those cases where a medicated spherical particle prepared using the spherical particle is incorporated within an orally rapid disintegrating tablet, problems such as a gritty sensation may occur when the tablet disintegrates inside the mouth. Accordingly, the particle size of the spherical particle is specified as not more than 250 $\mu$m.

Moreover, provided the sphericity (the ratio between the minor axis and the major axis) of the spherical particle is not less than 0.8, favorably flowability is obtained, including a reduced angle of repose. Furthermore, the closer the particle shape is to being spherical, the more uniform the layering is that is formed during the drug preparation process, meaning any variation in the amount of the active substance can be minimized.

Furthermore, the bulk density of the spherical particle of the present invention is typically not less than 0.6 g/ml, and is preferably 0.65 g/ml or greater. Provided the bulk density is not less than 0.6 g/ml, the flowability of the spherical particles is favorable, resulting in more favorable handling during drug preparation.

Method for producing spherical particle

The method for producing a spherical particle according to the present invention includes a granulation step of rolling a sugar alcohol having an average particle size of not more than 40 $\mu$m and a cellulose having an average particle size of not more than 50 $\mu$m while spraying a liquid thereon.

(Sugar alcohol)

Some varieties of sugar alcohol have a crystal shape that is needle-like or rod-shaped, and these crystals can cause a deterioration in the sphericity of the resulting spherical particle. Accordingly, in order to ensure a high degree of sphericity for the spherical particle, the sugar alcohol may be subjected to a grinding treatment to form a powder with the desired average particle size. Examples of methods that may be used for grinding the sugar alcohol include methods that employ an impact grinding mill or a jet grinding mill.

The sugar alcohol has an average particle size that is typically not more than 40 $\mu$m, preferably not more than 25 $\mu$m, and still more preferably 15 $\mu$m or less. Provided the average particle size of the sugar alcohol is not more than 40 $\mu$m, no unevenness is generated on the particle surface during granulation of the spherical particle, meaning a spherical particle having a high degree of sphericity can be obtained.

Furthermore, a sugar alcohol having an average particle size of not more than 40 $\mu$m can be dispersed uniformly through the spherical particle, facilitating a more uniform compositional ratio within the spherical particle.

(Cellulose)

The cellulose has an average particle size of not more than 50 $\mu$m, and the ratio between the major axis and the minor axis (hereafter also recorded as "L/D") is preferably not less than 1.5. In a particularly preferred configuration, the average particle size is not more than 25 $\mu$m, and L/D is 2.0 or greater.
Provided the average particle size of the cellulose is not more than 50 $\mu$m and the L/D ratio is at least 1.5, a spherical particle having a high degree of hardness, no unevenness on the particle surface, and a high degree of sphericity can be obtained when the spherical particle undergoes granulation. However, if granulation of the spherical particle is conducted using a cellulose for which the average particle size is greater than 50 $\mu$m and the L/D ratio is less than 1.5, then the granulation process proceeds without the cellulose incorporating the sugar alcohol uniformly therein, meaning the compositional ratio of the sugar alcohol and the cellulose within the granulated particle may lack uniformity. Accordingly, the L/D ratio of the cellulose is preferably not less than 1.5.

(Granulation step)

In the granulation step, granulation is preferably conducted via a method that uses, for example, a centrifugal rolling granulator.

In this type of method, first a centrifugal rolling granulator equipped with a rotating disc having aeration slits (such as the commercially available products "CF granulator" and "Granurex" manufactured by Freund Corporation.) is charged with the desired mass ratio of the powdered sugar alcohol and cellulose and the like. Subsequently, while the powders are subjected to centrifugal rolling inside the granulator under the actions of the centrifugal force generated by the rotating disc and the slit air, a liquid is sprayed onto the powders to form a wet granulated particle. During this method, the powders placed in the centrifugal rolling granulator may be already hydrated. Examples of methods of hydrating the powders include methods in which a predetermined amount of water is added to the powder inside a mixing granulator or a kneader or the like, and following kneading, is subjected to grinding using a mill such as a power mill.

The liquid that is sprayed onto the powders is preferably water or a mixture of water and an organic solvent such as ethanol.

In particular, if an alcohol such as ethanol is added to the water, then the amount of the sugar alcohol dissolved by the water can be reduced, which enables the development of adhesiveness to be suppressed during the granulation. Accordingly, if a comparison is made between the case where only water is spayed on the powders and the case where a mixture of water and an organic solvent such as ethanol is sprayed on the powders, then the case where a mixture of water and an organic solvent such as ethanol is sprayed on the powders tends to yield better suppression of powder adhesion to the granulator and better suppression of particle aggregation during the granulation process, meaning the yield of very fine spherical particles tends to increase. Moreover, when comparing the case where only water is spayed on the powders and the case where a mixture of water and an organic solvent such as ethanol is sprayed on the powders, the case where a mixture of water and an organic solvent such as ethanol is sprayed on the powders tends to also offer other advantages, including a reduction in the drying time of the spherical particles.

Furthermore, during the granulation step, an intermediate treatment is preferably conducted in which the rolling is continued while the spraying of the liquid is halted so that the granulation and drying occur simultaneously. This intermediate treatment is conducted for the purposes of regulating the water retention rate of the granulated particles and improving the sphericity during the granulation step.

In the granulation step, if the granulated particles become slightly dry, then the bulk density and the sphericity tend to decrease. However, on the other hand, if the granulated particles contain excess moisture, then the granulation process tends to progress to an excessive degree, and as a result, aggregates tend to form, granulated particles having a sharp particle size distribution can not be obtained, and the yield of particles having a particle size within a specified range tends to decrease. The extent of these problems is affected by the strong water retention properties of the cellulose, and in order to avoid these problems, it is important that the amount of water added during the granulation step is carefully controlled in order to enable the water retention rate of the granulated particles to be kept at a uniform level.

Accordingly in the present invention, during the granulation step that involves accumulating moisture on the particles by spraying with a liquid while the granulation proceeds, an intermediate treatment is preferably performed in which the spraying of the liquid is halted, and particle granulation and drying are allowed to proceed simultaneously using the accumulated moisture. Including this intermediate treatment facilitates control of the water retention rate (mainly control of the cellulose water retention rate) and control of the particle size. Accordingly, particularly in those cases where a granulated particle having a small particle size is being targeted, conducting this intermediate treatment enables the production of granulated particles having a uniform particle size, a high degree of hardness and a sphericity that is close to 1, without increasing the amount of aggregation.

Finally, the obtained granulated particles are dried to yield the spherical particles of the present invention.

The drying may be performed via an arbitrary method using a fluidized bed apparatus or a plate dryer or the like.

Effects

In the present invention, by employing a blend ratio of a sugar alcohol, that exhibits a low degree of reactivity with active substances and the like, and a cellulose, a spherical particle can be obtained which although being substantially soluble in water, having low reactivity and exhibiting superior disintegration properties in water, has a high degree of hardness and is resistant to abrasion. By using a spherical particle of the present invention having these properties in a medicated spherical particle, any reduction in the bioavailability can be prevented, the type of active substance used need not be restricted, and abrasion during drug preparation can also be prevented.

Furthermore, the production method of the present invention includes a granulation step in which granulation is conducted while a liquid is sprayed onto a sugar alcohol having an average particle size of not more than 40 $\mu$m and a cellulose having an average particle size of not more than 50 $\mu$m. Accordingly, a spherical particle is obtained that has a high degree of sphericity and for which the compositional ratio between the sugar alcohol and the cellulose is uniform.

Moreover, by performing an intermediate treatment during the granulation step, a spherical particle is obtained that exhibits an even higher degree of sphericity, a very sharp particle size distribution, and superior levels of hardness and bulk density and the like.

As described above, in the present invention, a spherical particle having favorable flowability and exhibiting minimal reactivity with active substances during drug preparation can be obtained with good yield. Furthermore, the spherical particle obtained in the present invention can be used to efficiently prepare a product in which the surface of the particle is layered with an active substance (namely, a medicated spherical particle).

EXAMPLES

Measurements and evaluations of the various physical properties of the spherical particles obtained in the examples and comparative examples were conducted using the methods described below. The results are listed in Table 1.

Bulk density

The spherical particles were placed inside a 100 ml measuring cylinder (mass: W) without any consolidation, and following level adjustment, the mass Wb of the filled cylinder was measured.

$$(Wb-W)/100 = d \qquad (1)$$

The value determined using formula (1) was the bulk density (d), and the average value of 5 measurements was calculated.

Sphericity

The sphericity is defined as the ratio between the minor axis and the major axis of the spherical particle, and the average ratio for 50 spherical particles was reported as the sphericity. Randomly sampled spherical particles were photographed, and for each of 50 spherical particles, the length of the major axis, and the length of the minor axis drawn perpendicularly from the midpoint of the major axis were measured. The ratio between the minor axis and the major axis (minor axis / major axis) was determined, and the average value was calculated for the 50 particles.

Average particle size

The average particle size was calculated using a Microtrac particle size distribution analyzer (9320HRA(X-100), manufactured by Nikkiso Co., Ltd.).

Example 1

First, an impact mill (ACM-10A, manufactured by Hosokawa Micron Corporation) was used to prepare D-mannitol with an average particle size of 15 $\mu$m.

Next, 2.4 kg of the prepared D-mannitol and 1.6 kg of a powdered cellulose (A) (average particle size: 24 $\mu$m, ratio between the major axis and minor axis (L/D): 3.1) were placed inside a centrifugal rolling granulator (Granurex GX-40, manufactured by Freund Corporation.) while supplying slit air to the granulator, and the rotating disc was rotated at a rate of 250 rpm.

Subsequently, granulation was conducted by spraying 1,900 g of water into the granulator at a rate of 10 to 40 g/min. During this process, at the points where 1,400 g and 1,600 g respectively of water had been sprayed, the water spraying was halted for a period of 10 minutes while the rolling of the granulated particles was continued (intermediate treatments). Once the spraying of the 1,900 g of water had been completed, the granulated particles were rolled for a further 30 minutes, yielding the product granulated particles via a total of three intermediate treatments.

Finally, the obtained granulated particles were transferred to a fluidized bed granulation coating apparatus (Flow Coater FLO-5, manufactured by Freund Corporation.), a heated air stream set to a temperature of 90°C was introduced into the fluidized bed apparatus, and drying was conducted until the temperature of the granulated particles reached 60°C.

As a result, spherical particles having a sphericity of 0.84, a bulk density of 0.70 g/ml, and a particle size of 75 to 150 $\mu$m were obtained with a yield of 80%.

Example 2

First, D-mannitol with an average particle size of 15 μm was prepared in the same manner as example 1. Subsequently, 0.9 kg of the prepared D-mannitol and 0.6 kg of a crystalline cellulose (B) (average particle size: 20 μm, L/D: 2.9) were placed inside a kneader, 300 g of water was added, and the mixture was kneaded for 20 minutes. The resulting product was ground using a power mill, yielding a wet powder.

Next, 1.5 kg of this wet powder was placed inside a centrifugal rolling granulator (CF Granulator CF-360N, manufactured by Freund Corporation.) while supplying slit air to the granulator, and the rotating disc was rotated at a rate of 250 rpm. Subsequently, granulation was conducted by spraying 800 g of a mixed solvent of water : ethanol = 1:1 (volumetric ratio) into the granulator at a rate of 10 to 40 g/min. During this process, at the points where 420 g and 700 g respectively of the mixed solvent had been sprayed, the spraying of the mixed solvent was halted for a period of 10 minutes while the rolling of the granulated particles was continued (intermediate treatments). Once the spraying of the 800 g of the mixed solvent had been completed, the granulated particles were rolled for a further 30 minutes, yielding the product granulated particles via a total of three intermediate treatments.

Finally, the obtained granulated particles were dried in the same manner as example 1.

As a result, spherical particles having a sphericity of 0.83, a bulk density of 0.68 g/ml, and a particle size of 75 to 150 μm were obtained with a yield of 83%.

Example 3

First, xylitol with an average particle size of 15 μm was prepared using the same method as that described in example 1.

Subsequently, 0.75 kg of the prepared xylitol and 0.75 kg of the powdered cellulose (A) (average particle size: 24 μm, L/D: 3.1) were placed inside a centrifugal rolling granulator (CF-360N) while supplying slit air to the granulator, and the rotating disc was rotated at a rate of 250 rpm.

Subsequently, granulation was conducted by spraying 600 g of water into the granulator at a rate of 10 to 40 g/min. During this process, at the points where 350 g and 520 g respectively of water had been sprayed, the water spraying was halted for a period of 10 minutes while the rolling of the granulated particles was continued (intermediate treatments). Once the spraying of the 600 g of water had been completed, the granulated particles were rolled for a further 30 minutes, yielding the product granulated particles via a total of three intermediate treatments.

Finally, the obtained granulated particles were dried in the same manner as example 1.

As a result, spherical particles having a sphericity of 0.84, a bulk density of 0.68 g/ml, and a particle size of 75 to 150 μm were obtained with a yield of 82%.

Example 4

First, D-sorbitol with an average particle size of 15 μm was prepared using the same method as that described in example 1.

Subsequently, 2.8 kg of the prepared D-sorbitol and 1.2 kg of a powdered cellulose (C) (average particle size: 28 μm, L/D: 3.3) were placed inside a centrifugal rolling granulator (GX-40) while supplying slit air to the granulator, and the rotating disc was rotated at a rate of 250 rpm.

Subsequently, granulation was conducted by spraying 1,600 g of water into the granulator at a rate of 10 to 40 g/min. During this process, at the points where 1,100 g and 1,450 g respectively of water had been sprayed, the water spraying was halted for a period of 10 minutes while the rolling of the granulated particles was continued (intermediate treatments). Once the spraying of the 1,600 g of water had been completed, the granulated particles were rolled for a further 30 minutes, yielding the product granulated particles via a total of three intermediate treatments.

Finally, the obtained granulated particles were dried in the same manner as example 1.

As a result, spherical particles having a sphericity of 0.85, a bulk density of 0.71 g/ml, and a particle size of 150 to 250 μm were obtained with a yield of 80%.

Example 5

First, the same D-mannitol and powdered cellulose (A) as those used in example 1 were placed inside a centrifugal rolling granulator (GX-40) while supplying slit air to the granulator, and the rotating disc was rotated at a rate of 250 rpm.

Subsequently, granulation was conducted by spraying 1,900 g of water into the granulator at a rate of 10 to 30 g/min, yielding granulated particles.

Finally, the obtained granulated particles were dried in the same manner as example 1.

As a result, many of the obtained spherical particles had a particle size of 150 µm or greater, although spherical particles having a sphericity of 0.82, a bulk density of 0.69 g/ml, and a particle size of 75 to 150 µm were obtained with a yield of 59%.

Comparative example 1

First, 3 kg of D-mannitol with an average particle size of 50 µm was placed inside a centrifugal rolling granulator (CF-360N) while supplying slit air to the granulator, and the rotating disc was rotated at a rate of 250 rpm.
Subsequently, granulation was conducted by spraying 820 g of a 20% by mass aqueous solution of D-mannitol into the granulator at a rate of 5 to 15 g/min, yielding granulated particles.
Finally, the obtained granulated particles were dried in the same manner as example 1.
As a result, the granulated product included ungranulated fine powder and a large quantity of aggregates, and spherical particles having a sphericity of 0.82, a bulk density of 0.62 g/ml and a particle size of 75 to 150 µm, which although being spherical also exhibited significant surface unevenness, were obtained with a yield of 42%.

Comparative example 2

First, 3 kg of lactose with an average particle size of 50 µm was placed inside a centrifugal rolling granulator (CF-360N) while supplying slit air to the granulator, and the rotating disc was rotated at a rate of 250 rpm.
Subsequently, granulation was conducted by spraying 480 g of water into the granulator at a rate of 10 g/min, yielding granulated particles.
Finally, the obtained granulated particles were dried in the same manner as example 1.
As a result, spherical particles having a sphericity of 0.81, a bulk density of 0.70 g/ml, and a particle size of 75 to 150 µm were obtained with a yield of 71%.

Comparative example 3

First, 2.4 kg of D-mannitol with an average particle size of 50 µm and 1.6 kg of a crystalline cellulose (D) (average particle size: 50 µm, L/D: 1.4) were placed inside a centrifugal rolling granulator (GX-40) while supplying slit air to the granulator, and the rotating disc was rotated at a rate of 250 rpm.
Subsequently, granulation was conducted by spraying 1,900 g of water into the granulator at a rate of 30 to 40 g/min, yielding granulated particles.
Finally, the obtained granulated particles were dried in the same manner as example 1.
As a result, the granulated product included a large number of particles with a particle size of 150 µm or greater and a large amount of rod-shaped particles, and spherical particles having a sphericity of 0.73, although suffering from noticeable surface unevenness, a bulk density of 0.60 g/ml and a particle size of 75 to 150 µm were obtained with a yield of 55%.

[Table 1]

| Powder | Average particle size (μm) | Example 1 | Example 2 | Example 3 | Example 4 | Example 5 | Comparative example 1 | Comparative example 2 | Comparative example 3 |
|---|---|---|---|---|---|---|---|---|---|
| D-mannitol (kg) | 15 | 2.4 | 0.9 | | | 2.4 | | | |
| Xylitol (kg) | 15 | | | 0.75 | | | | | |
| D-sorbitol (kg) | 15 | | | | 2.8 | | | | |
| D-mannitol (kg) | 50 | | | | | | 3 | | 2.4 |
| Lactose (kg) | 50 | | | | | | | 3 | |
| Powdered cellulose (A) (kg) (L/D: 3.1) | 24 | 1.6 | | 0.75 | | 1.6 | | | |
| Crystalline cellulose (B) (kg) (L/D: 2.9) | 20 | | 0.6 | | | | | | |
| Powdered cellulose (C) (kg) (L/D: 3.3) | 28 | | | | 1.2 | | | | |
| Crystalline cellulose (D) (kg) (L/D: 1.4) | 50 | | | | | | | | 1.6 |
| Water (kg) | | 1.9 | 0.3 | 0.6 | 1.6 | 1.9 | | 0.48 | 1.9 |
| Water / ethanol (kg) (1:1) | | | 0.8 | | | | | | |
| 20% D-mannitol aqueous solution (kg) | | | | | | | 0.82 | | |
| Yield (%) (average particle size: 75 to 150 μm) | | 80 | 83 | 82 | | 59 | 42 | 71 | 55 |
| Yield (%) (average particle size: 150 to 250 μm) | | | | | 80 | | | | |
| Bulk density (g/ml) | | 0.70 | 0.68 | 0.68 | 0.71 | 0.69 | 0.62 | 0.70 | 0.60 |
| Sphericity | | 0.84 | 0.83 | 0.84 | 0.85 | 0.82 | 0.82 | 0.81 | 0.73 |

* L/D: ratio between the major axis and the minor axis

The spherical particles obtained in the examples had particle sizes within a range from 75 to 250 μm, and exhibited excellent levels of bulk density, sphericity and surface state, and therefore the flowability required during drug preparation was able to be ensured. Moreover, by appropriate adjustment of the blend ratio between the sugar alcohol and the cellulose, spherical particles were obtained that were substantially soluble in water, and exhibited superior versatility in terms of use with all manner of active substances (medications).

In contrast, the spherical particles of comparative example 1 not only contained no cellulose, but were also granulated without performing an initial grinding step, using only a water-soluble component having a large particle size and without conducting an intermediate treatment, and as a result, the yield of spherical particles having a particle size of 75 to 250 μm was low, and unevenness existed on the particle surfaces.

The spherical particles of comparative example 2 were formed solely from lactose, which tends to be prone to interaction with active substances (medications), and therefore suffered from poor versatility relative to active substances (medications) (see test example 1).

In comparative example 3, the spherical particles were prepared from a large particle size D-mannitol that had not undergone grinding and a crystalline cellulose having a small L/D ratio, and the desired surface state and sphericity were unobtainable.

Test example 1

Using the granulated particles of 75 to 150 μm obtained in example 1 and comparative example 2 as core particles, medicated spherical particles were prepared containing 50% by mass of aminophylline as a pharmaceutical active substance.
100 g of each of these medicated spherical particles was placed in a sample bottle and sealed, and the sample was stored at 60°C. Each of the stored samples was tested for storage stability using the method outlined below. The results are listed in Table 2.
Color difference
Based on the L*a*b* color system, a color-difference meter (SZ-Σ90, manufactured by Nippon Denshoku Industries Co., Ltd.) was used to calculate the color difference ΔE (in accordance with JIS Z-8729 and 8730).

[Table 2]

|  | Example 1 | | Comparative example 2 | |
|---|---|---|---|---|
|  | Change in coloration (ΔE) | Color | Change in coloration (ΔE) | Color |
| 3 days (60°C) | 3.0 | White | 72.0 | Brown |
| 7 days (60°C) | 4.1 | White | 72.3 | Brown |

The medicated particles that used the spherical particles of example 1 formed from D-mannitol and cellulose as the core particles remained white after both 3 days and 7 days, and displayed minimal change in coloration. In contrast, the medicated particles that used the spherical particles of comparative example 2 formed from lactose as the core particles had turned brown after 3 days as a result of a Maillard reaction between the aminophylline and the lactose, resulting in a dramatic change in coloration.
While preferred embodiments of the invention have been described and illustrated above, the present invention is in no way limited by these embodiments. Additions, omissions, substitutions, and other modifications can be made without departing from the scope of the present invention. Accordingly, the invention is not to be considered as being limited by the foregoing description, and is only limited by the scope of the appended claims.

INDUSTRIAL APPLICABILITY

The present invention relates to a spherical particle that can be used in orally rapid disintegrating tablets and capsules. The spherical particle of the present invention is obtained via a granulation step of rolling a sugar alcohol having an average particle size of not more than 40 μm and a crystalline cellulose and/or powdered cellulose having an average particle size of not more than 50 μm while spraying a liquid thereon. In the spherical particle of the present invention, the mass ratio between the sugar alcohol and the crystalline cellulose and/or powdered cellulose is within a range from 50:50 to 90:10, the particle size is within a range from 75 to 250 μm, the sphericity (minor axis / major axis) is not less than 0.8, and the bulk density is not less than 0.6 g/ml. Accordingly, when used in the drug preparation of an orally rapid disintegrating tablet or a capsule, the spherical particle of the present invention offers the advantages of a high degree of versatility that does not restrict the active substances that can be used, favorable resistance to abrasion, and minimal reduction in bioavailability.

Claims

1. A spherical particle, comprising a sugar alcohol, and a crystalline cellulose and/or powdered cellulose, wherein

a mass ratio between the sugar alcohol and the crystalline cellulose and/or powdered cellulose is within a range from 50:50 to 90:10,
particle size is within a range from 75 to 250 μm, sphericity (minor axis / major axis) is not less than 0.8, and bulk density is not less than 0.6 g/ml.

2.  A spherical particle according to claim 1, wherein the sugar alcohol is D-mannitol, xylitol, D-sorbitol or erythritol.

3.  A spherical particle according to claim 1 or claim 2, wherein the sugar alcohol is D-mannitol. 1.

4.  A method for producing a spherical particle, the method comprising
    a granulation step of rolling a sugar alcohol having an average particle size of not more than 40 μm and a crystalline cellulose and/or powdered cellulose having an average particle size of not more than 50 μm while spraying a liquid thereon.

5.  A method for producing a spherical particle according to claim 4, wherein the sugar alcohol is in a ground form.

6.  A method for producing a spherical particle according to claim 4 or claim 5, wherein, in the granulation step, an intermediate treatment is conducted in which rolling is performed while spraying of the liquid is halted.

## INTERNATIONAL SEARCH REPORT

| International application No. |
| --- |
| PCT/JP2008/070888 |

A. CLASSIFICATION OF SUBJECT MATTER
*A61K47/10*(2006.01)i, *A61K9/16*(2006.01)i, *A61K47/38*(2006.01)i

According to International Patent Classification (IPC) or to both national classification and IPC

B. FIELDS SEARCHED

Minimum documentation searched (classification system followed by classification symbols)
A61K47/10, A61K9/16, A61K47/38

Documentation searched other than minimum documentation to the extent that such documents are included in the fields searched
Jitsuyo Shinan Koho        1922-1996    Jitsuyo Shinan Toroku Koho    1996-2008
Kokai Jitsuyo Shinan Koho    1971-2008    Toroku Jitsuyo Shinan Koho    1994-2008

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
| --- | --- | --- |
| A | JP 61-213201 A  (Fuji Paudal Co., Ltd.), 22 September, 1986 (22.09.86), Full text (Family: none) | 1-6 |
| A | JP 5-229961 A  (Freund Industrial Co., Ltd.), 07 September, 1993 (07.09.93), Full text (Family: none) | 1-6 |
| A | JP 4-283520 A  (Asahi Chemical Industry Co., Ltd.), 08 October, 1992 (08.10.92), Full text (Family: none) | 1-6 |

[X]  Further documents are listed in the continuation of Box C.        [ ]  See patent family annex.

| | |
| --- | --- |
| *    Special categories of cited documents: <br> "A"  document defining the general state of the art which is not considered   to be of particular relevance <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search <br>     04 December, 2008 (04.12.08) | Date of mailing of the international search report <br>     16 December, 2008 (16.12.08) |
| --- | --- |
| Name and mailing address of the ISA/ <br>     Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2008/070888

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2001-172430 A  (Asahi Kasei Corp.),<br>26 June, 2001 (26.06.01),<br>Full text<br>(Family: none) | 1-6 |
| A | WO 02/002643 A1  (Asahi Kasei Kabushiki Kaisha),<br>10 January, 2002 (10.01.02),<br>Full text<br>& US 2004/0053887 A1    & EP 1300420 A1 | 1-6 |
| A | EP 452862 A2  (Asahi Chemical Industry Co., Ltd.),<br>23 October, 1991 (23.10.91),<br>Full text<br>& JP 7-173050 A         & US 5384130 A<br>& US 5505983 A | 1-6 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007299546 A
- JP HEI9295947 B
- JP HEI920689 B
- JP HEI7173050 B